Europäisches Patentamt

European Patent Office

Office européen des brevets

(19)

(11) Veröffentlichungsnummer: **0 231 229**
**B1**

(12) **EUROPÄISCHE PATENTSCHRIFT**

(45) Veröffentlichungstag der Patentschrift: **16.01.91**

(21) Anmeldenummer: **86904152.5**

(22) Anmeldetag: **04.07.86**

(86) Internationale Anmeldenummer:
**PCT/EP86/00394**

(87) Internationale Veröffentlichungsnummer:
**WO 87/00416 29.01.87 Gazette 87/03**

(51) Int. Cl.⁵: **A 61 B 5/20, A 61 B 8/00**

(54) GERÄT ZUR BESTIMMUNG DES FÜLLGRADES DER MENSCHLICHEN BLASE.

(30) Priorität: **22.07.85 DE 3526164**

(43) Veröffentlichungstag der Anmeldung:
**12.08.87 Patentblatt 87/33**

(45) Bekanntmachung des Hinweises auf die
Patenterteilung:
**16.01.91 Patentblatt 91/03**

(84) Benannte Vertragsstaaten:
**AT BE CH DE FR GB IT LI LU NL SE**

(56) Entgegenhaltungen:
**US-A-3 672 352**
**US-A-4 109 644**

**IEEE Transactions on Biomedical Engineering, volume BME-26, No. 12, December 1979, (New York, US), Rise et al: "A ultrasonic bladder-volume sensor", pages 709-711,**

(73) Patentinhaber: **MÜLLER-WICKOP, Jürgen**
**Marderstieg 2**
**D-2000 Hamburg 65 (DE)**

(72) Erfinder: **MÜLLER-WICKOP, Jürgen**
**Marderstieg 2**
**D-2000 Hamburg 65 (DE)**

(74) Vertreter: **Glawe, Delfs, Moll & Partner**
**Patentanwälte**
**Postfach 26 01 62 Liebherrstrasse 20**
**D-8000 München 26 (DE)**

(56) References cited:
**IEEE Transactions on Biomedical Engineering, volume BME 19, No. 3, May 1972, (New York, US), Dreher et al: "Bladder volume sensing by local distension measurement", pages 247-248 Radiology, volume 128, July 1978, MacLean et al: "Determination of bladder volumes by gray scale ultrasonography", pages 181-182**

Courier Press, Leamington Spa, England.

EP 0 231 229 B1

## Beschreibung

Die Erfindung betrifft ein in den menschlichen Körper implantierbares Gerät, zur Bestimmung des Füllgrades den menschlichen Blase, das einen Ultraschallgeber, einen Ultraschallempfänger, eine elektrische Schaltung zur Erregung des Ultraschallgebers und zum Empfangen und Auswerten der Signale des Ultraschallempfängers sowie eine Meldeeinrichtung aufweist wobei die Laufkeit des Ultraschallpulses zwischen Ultraschallgeben und Empfangen ein maß ist für den Füllgrad der Blase.

Ein gesunder Mensch bemerkt rechtzeitig, wenn seine Blase so weit gefüllt ist, daß sie entleert werden sollte. Dies kann bei bestimmten Erkrankungen (z.B. Querschnittlähmung, multipler Sklerose oder Tumoren) nicht mehr der Fall sein. Entleeren diese Kranken aber aufgrund der Tatsache, daß sie die Füllung der Blase nicht bemerken, dieselbe nicht rechtzeitig oder vollständig, so besteht die Gefahr einer Niereninfektion, da Urin in die Nieren zurückströmen kann. Dies kann durch chronische Nierenentzündungen zum Nierenverlust führen. Auch besteht die Gefahr einer ungewollten und unkontrollierten Entleerung der Blase, wenn diese nicht rechtzeitig in kontrollierter und gewollter Weise z.B. durch Beklopfen der unteren Bauchwand entleert wird.

Aus dem Artikel von W. Bleifeld und S. Effert, "Über den Nachweis zystischer Gebilde in verschiedenen Körperregionen mit dem Ultraschallreflexionsverfahren", Deutsche Medizinische Wochenschrift, 92. Jahrgang, Nr. 39, Seite 1747 bis 1750 (1967) ist es bekannt, zystische, d.h. mit Körperflüssigkeit gefüllte Gebilde im menschlichen Körper festzustellen. Zu diesem Zweck wird ein Ultraschallgeber auf die menschliche Haut gesetzt und das Echo von Ultraschallimpulsen aufgezeichnet. Diese Ultraschallechos erscheinen normalerweise für eine gewisse Zeit als eine mehr oder weniger ununterbrochene Folge von Signalen, da der Ultraschall überall im menschlichen Körper wenigstens teilweise reflektiert wird. Dringt der Ultraschall aber an einer Stelle in den menschlichen Körper ein, unter der sich eine Zyste befindet, so tritt für eine gewisse Zeit, die der Laufzeit des Ultraschallpulses durch die homogene Flüssigkeit der Zyste entspricht, kein Ultraschallecho auf. Auf diese Weise kann die Anwesenheit der Zyste sowie ihr Durchmesser festgestellt werden.

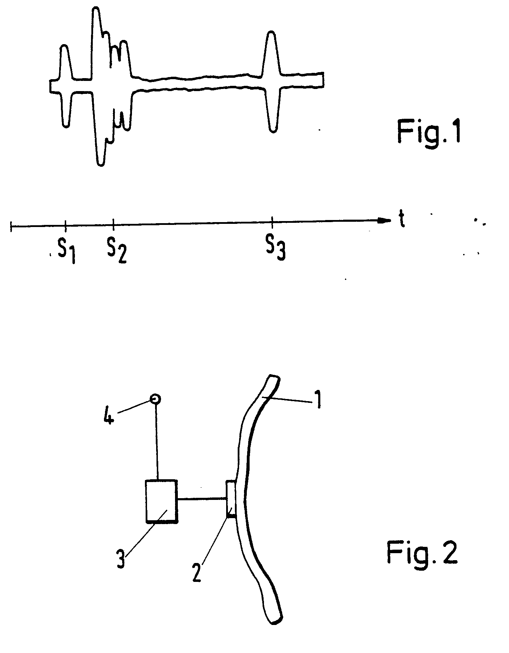

Es ist bekannt, auch den Füllgrad der menschlichen Blase mit Hilfe von Ultraschall zu bestimmen. Bei einem vorbekannten Verfahren (IEEE Transactions on Biomedical Engineering, Band BME-26, Nr. 12, December 1979, (New York, US), Rise et al.: "A ultrasonic bladder-volume sensor", Seiten 709—711, siehe Seiten 709—710, Abschnitte 'Introduction'', "Method and materials'', "Results''; Figur 1). geschieht die Messung durch ein Gerät, das außen auf den Körper aufgesetzt wird. Dies ist offenbar auch bei einem anderen Verfahren (Radiology, Band 128, Juli 1978, MacLean et al.: "Determination of bladder volumes by gray scale ultrasonography", siehe Seiten 181—182) der Fall, obwohl die Entgegenhaltung hierzu keine weiteren Angaben enthält.

Die Erfindung geht von der Erkenntnis aus, daß die Bestimmung des Füllgrads auch mit einem implantierbaren Gerät der eingangs genannten Art (US—PS—4 109 644) vorgenommen werden kann. Der Vorteil dieses Gerätes besteht darin, daß zwar ein Teil dauernd im Körper implantiert ist, so daß nicht zunächst nach Absenden des Ultraschallpulses Echos auftreten, die dem Laufweg des Ultraschallpulses durch die Bauchdecke bis zur Blasenwand entsprechend, wodurch nicht nur die Signalauswertung komplizierter würde, sondern auch das Signal abgeschwächt würde. Der Nachteil besteht aber immer noch darin, daß die Messung bzw. Bestimmung des Füllgrades durch ein separates Gerät von außen erfolgen muß, an das Signale vom implantierten Gerät drahtlos übertragen werden.

Die Aufgabe der Erfindung besteht in der Schaffung eines implantierbaren Geräts, bei dem der Patient ohne äußere Geräte oder Geräteteile merkt, daß seine Blase einen bestimmten Füllgrad erreicht hat.

Die erfindungsgemäße Lösung besteht darin, daß die Meldeeinrichtung eine in die Haut einpflanzbare Elektrode ist.

Diese Elektrode setzt bei Erreichen eines bestimmten Füllgrades in der Haut einen elektrischen Reiz, so daß der Patient merkt, daß seine Blase einen bestimmten Füllgrad erreicht hat. Vorteilhafter ist es aber, wenn zwei Elektroden vorgesehen sind, da dann mehr Information gemeldet werden kann. Man könnte zum Beispiel vorsehen, daß bei einem verhältnismäßig geringen Füllgrad eine Elektrode, bei einem größeren Füllgrad die andere Elektrode und schließlich bei sehr starker Füllung beide Elektroden ein Reizsignal abgeben. Dabei werden die Elektroden in Bereichen eingepflanzt, in denen die Haut noch sensibel ist. Die Verbindung mit dem Gerät kann dann durch ein subcutanes Kabel erfolgen.

Wie erwähnt treten, da das Gerät in den menschlichen Körper implantierbar ist, die Probleme der Ultraschallreflektion an der Bauchwand nicht mehr auf. Startsignal des Ultraschallpulses, erstes Blasenwandecho, echofreie Strecke und zweites Blasenwandecho sind dann auch eindeutig identifizierbar und mit Verfahren der automatischen Mustererkennung auswertbar. Weiter muß die Blase für die Messung nicht mehr mühsam von außen geortet werden. Ein weiterer Vorteil ist, daß wegen der Anwesenheit der Serosaflüssigkeit keine besondere Flüssigkeit verwendet werden muß, um den Schallübergang vom Ultraschallgeber/empfänger zum Gewebe zu gewährleisten. Schließlich trägt der Patient das Gerät ständig mit sich herum, ohne daß es in störender Weise außen am Körper befestigt ist.

Vorteilhafterweise sind Ultraschallgeber und -empfänger als eine Einheit ausgebildet, die dabei insbesondere einen piezoelektrischen Kristall aufweisen kann. Auf diese Weise erhält man ein wesentlich einfacheres Gerät, als wenn man Ultraschallgeber und -empfänger als getrennte Einheiten vorsieht.

Der Ultraschallgeber/empfänger kann dabei insbesondere auf oder in der Blasenwand befestigt werden, da in diesem Falle die Ultraschallwellen nur einen sehr kleinen Weg (die Dicke der Blasenwand) durchlaufen müssen, in dem störende Reflektionen auftreten können. Man erhält so ein verhältnismäßig scharfes Startsignal, so daß die Zeit bis zum Eintreffen des Echos von der gegenüberliegenden Blasenwand und damit der Füllgrad genau bestimmt werden kann. Dabei kann der Ultraschallgeber/empfänger z.B. aufgenäht, aufgeklebt oder in eine Fascientasche eingenäht werden.

Wenn mehrere Ultraschallgeber/empfänger vorgesehen sind, kann die Genauigkeit erhöht werden. Zum Beispiel könnten die Signale dieser mehreren Ultraschallgeber/empfänger miteinander verglichen werden.

Besonders vorteilhaft ist es, wenn drei Ultraschallgeber/empfänger vorgesehen sind, insbesondere, wenn dieselben so angebracht sind, daß sie die Blasenausdehnung in drei im wesentlichen zueinander senkrechten Richtungen bestimmen. In diesem Falle kann sehr genau das Volumen des Blaseninhalts bestimmt werden.

Die Energieversorgung des Geräts kann mit Batterien erfolgen, die ebenfalls innerhalb des Körpers angeordnet sind. Besonders zweckmäßig ist es, wenn das Gerät einen aufladbaren Akkumulator aufweist. Dabei ist es besonders vorteilhaft, wenn das Gerät eine Einrichtung zum Aufladen des Akkumulators von außen mit Hilfe von Induktion aufweist, da dann eine elektrisch leitende Verbindung zwischen dem Gerät im Körperinneren und dem Ladegerät nicht hergestellt werden muß.

Zweckmäßigerweise weist das Gerät zum Auswerten der Signale einen Mikroprozessor auf.

Zweckmäßigerweise weist das Gerät eine Zeitschaltung zum Messen und Übermitteln der Füllgradinformation in regelmäßigen Zeitabständen auf, da auf diese Weise Energie gespart wird. Bei transcutaner Messung besteht aber eine etwas andere Zielsetzung, so daß dort kontinuierlich bzw. zu einem wählbaren Zeitpunkt gemessen wird oder die Zeitabstände transcutan veränderbar sind.

Die Erfindung wird im folgenden anhand einer vorteilhaften Ausführungsform unter Bezugnahme auf die beigefügte Zeichnung beschrieben. Es zeigen in schematischer Darstellung:

Fig. 1 das Signal eines auf die Haut aufgesetzten Ultraschallempfängers nach Aussenden eines Ultraschallpulses, wenn innerhalb des Signalweges der Ultraschallpulse eine mit Flüssigkeit gefüllte Kammer im menschlichen Körper vorhanden ist; und

Fig. 2 den schematischen Aufbau des erfindungsgemäßen Gerätes.

In Fig. 1 ist die Signalform dargestellt, die auftritt, wenn man bei dem vorbekannten Verfahren mit Hilfe eines auf die Haut aufgesetzten Ultraschallgebers/Empfängers eine mit Flüssigkeit gefüllte Zyste feststellt, die sich im Wege der Ultraschallsignale befindet.

Das auf eines Oszillographen sichtbar gemachte Signal der Fig. 1 zeigt zunächst bei $S_1$ das Signal des ursprünglich ausgesendeten Ultraschallpulses. Bei $S_2$ treten dann eine große Zahl von Reflektionen auf, die dem Laufweg des Ultraschallpulses zwischen dem Sender durch die Haut und Bauchdecke bis zur Vorderwand der Zyste entsprechen. Anschliessend erfolgt über eine längere Zeitdauer kein Signal, da in dieser Zeit der Ultraschallpuls die homogene Flüssigkeit durchläuft, so daß hier keine Reflektionen stattfinden. Reflektionen treten erst auf, wenn der Ultraschallpuls die Rückwand der Zyste erreicht und hier teilweise reflektiert wird bzw. teilweise durch die Wand hindurchtritt und in seiner ursprünglichen Richtung weiterläuft. Das entsprechende Echo ist bei $S_3$ gezeigt.

Der Durchmesser der Zyste kann dabei aus dem Zeitraum bestimmt werden, während dem zwischen den Signalen $S_2$ und $S_3$ keine bzw. kaum Reflektionssignale auftreten.

Beim erfindungsgemäßen Gerät ist nun das Signal, das dem Signal $S_2$ entspricht, wesentlich zeitlich zusammengerückt, da der Ultraschall nicht mehr durch die Bauchdecke hindurchgehen muß. Ist insbesondere der Ultraschallgeber/empfänger auf die Blasenwand aufgenäht, so hat das Signal, das dem Signal $S_2$ entspricht, eine sehr kleine zeitliche Ausdehnung, so daß die Signaldauer ohne Echo zwischen den beiden Echos von der vorderen und hinteren Blasenwand sehr genau bestimmt werden kann.

Wie dies in Fig. 2 gezeigt ist, weist das Gerät einen auf die Blasenwand 1 aufgenähten Ultraschallgeber/Empfänger auf, der zum Beispiel ein piezoelektrischer Kristall sein kann. Dieser Ultraschallgeber/empfänger 2 ist mit einer elektronischen Schaltung 3 verbunden, die den Ultraschallgeber 2 mit Energie zur Erzeugung der Ultraschallpulse versorgt und die Reflektionssignale vom Ultraschallempfänger 2 empfängt, verstärkt und auswertet. Die Schaltung 3 gibt dann die Füllstandsinformation an eine Meldeeinrichtung 4 weiter, die aus einer oder mehreren Elektroden besteht und die in die Haut eingesetzt sind.

Zweckmäßigerweise ist die Schaltung 3 mit einem wieder aufladbaren Akku versehen, der durch die Haut hindurch induktiv aufgeladen werden kann.

**Patentansprüche**

1. In den menschlichen Körper implantierbares Gerät zur Bestimmung des Füllgrades der menschlichen Blase, das einen Ultraschallgeber (2), einen Ultraschallempfänger (2), eine elektrische Schaltung (3) zur Erregung des Ultraschallgebers (2) und zum Empfangen und Auswerten der Signale des Ultraschallempfängers (2) sowie eine Meldeeinrichtung (4) aufweist, wobei die Laufzeit des Ultraschallpulses zwischen ultraschallgeben und Empfangen ein maß ist für den Füllgrad der Blase und wobei die Meldeeinrichtung (4) eine in die Haut einpflanzbare Elektrode ist.

2. Gerät nach Anspruch 1, dadurch gekennzeichnet, daß zwei Elektroden vorgesehen sind.

3. Gerät nach Anspruch 1 oder 2, dadurch gekennzeichnet, daß Ultraschallgeber und -empfänger als eine Einheit (2) ausgebildet sind.

4. Gerät nach Anspruch 3, dadurch gekennzeichnet, daß der Ultraschallgeber/empfänger (2) einen piezoelektrischen Kristall aufweist.

5. Gerät nach einem der Ansprüche 1 bis 4, dadurch gekennzeichnet, daß mehrere Ultraschallgeber/empfänger (2) vorgesehen sind.

6. Gerät nach Anspruch 5, dadurch gekennzeichnet, daß es drei Ultraschallgeber/empfänger (2) aufweist.

7. Gerät nach einem der Ansprüche 1 bis 6, dadurch gekennzeichnet, daß es einen von außen mit Hilfe von Induktion aufladbaren Akkumulator enthält.

8. Gerät nach einem der Ansprüche 1 bis 7, dadurch gekennzeichnet, daß es einen Mikroprozessor aufweist.

9. Gerät nach einem der Ansprüche 1 bis 8, dadurch gekennzeichnet, daß es mit einer Zeitschaltung für das Messen und Übermitteln der Meldung in regelmäßigen Zeitabständen versehen ist.

**Revendications**

1. Appareil à implanter dans le corps humain pour déterminer le degré de remplissage de la vessie humaine, comportant un générateur d'ultrasons (2), un détecteur d'ultrasons (2), un circuit électrique (3) pour l'excitation du générateur d'ultrasons (2) et pour la réception et l'exploitation des signaux émis par le détecteur (2), ainsi qu'un dispositif indicateur (4), dans lequel la durée de propagation de l'impulsion ultrasonore entre le générateur d'ultrasons et le détecteur constitue une mesure du degré de remplissage de la vessie et dans lequel le dispositif indicateur (4) est une électrode implantable dans le peau.

2. Appareil selon la revendication 1, caractérisé en ce que deux électrodes sont prévues.

3. Appareil selon l'une ou l'autre des revendications 1 et 2, caractérisé en ce que le générateur et le détecteur d'ultrasons sont conçus sous la forme d'une unité (2).

4. Appareil selon la revendication 3, caractérisé en ce que le générateur/détecteur d'ultrasons (3) comporte un cristal piézoélectrique.

5. Appareil selon l'une quelconque des revendications 1 à 4, caractérisé en ce que plusieurs générateurs/détecteurs d'ultrasons (2) sont prévus.

6. Appareil selon la revendication 5, caractérisé en ce qu'il comporte trois générateurs/détecteurs d'ultrasons (2).

7. Appareil selon l'une quelconque des revendications 1 à 6, caractérisé en ce qu'il comporte un accumulateur chargeable par induction de l'extérieur.

8. Appareil selon l'une quelconque des revendications 1 à 7, caractérisé en ce qu'il comporte un microprocesseur.

9. Appareil selon l'une quelconque des revendications 1 à 8, caractérisé en ce qu'il est muni d'une minuterie pour la mesure et la transmission des informations à intervalles de temps réguliers.

**Claims**

1. An apparatus for determining the filling level of the human bladder, which can be implanted in the human body and which has an ultrasonic transmitter (2), an ultrasonic receiver (2), an electrical circuit (3) for energising the ultrasonic transmitter (2) and for receiving and evaluating the signals of the ultrasonic receiver (2), and also a warning device (4), wherein the transit time of the ultrasonic pulse between the ultrasonic transmitter and receiver is an indication of the filling level of the bladder and wherein the warning device (4) is an electrode which can be implanted in the skin.

2. An apparatus according to Claim 1, characterised in that two electrodes are provided.

3. An apparatus according to Claim 1 or 2, characterised in that the ultrasonic transmitter and receiver (2) are in the form of a unit (2).

4. An apparatus according to Claim 3, characterised in that the ultrasonic transmitter/receiver (2) has a piezoelectric crystal.

5. An apparatus according to any one of Claims 1 to 4, characterised in that a plurality of ultrasonic transmitter/receivers (2) are provided.

6. An apparatus according to Claim 5, characterised in that it has three ultrasonic transmitter/receivers (2).

7. An apparatus according to any one of Claims 1 to 6, characterised in that it includes a battery which can be charged from the outside by induction.

8. An apparatus according to any one of Claims 1 to 7, characterised in that it has a microprocessor.

9. An apparatus according to any one of Claims 1 to 8, characterised in that it is provided with a timing circuit for measuring and transmitting the warning at regular intervals.

Fig.1

$S_1$  $S_2$  $S_3$  $t$

Fig. 2